# EUROPÄISCHE PATENTSCHRIFT

(11) **EP 3 797 805 B1**
(45) Veröffentlichungstag und Bekanntmachung des Hinweises auf die Patenterteilung: **13.09.2023**
(21) Anmeldenummer: 20197901.0
(22) Anmeldetag: 23.09.2020
(51) Int. Cl.: A61M 1/36

(54) **BLUTBEHANDLUNGSVORRICHTUNG MIT AUTOMATISCHER LUFTENTFERNUNG**
BLOOD TREATMENT DEVICE WITH AUTOMATIC AIR REMOVAL
DISPOSITIF DE TRAITEMENT DU SANG À ÉLIMINATION AUTOMATIQUE DE L'AIR

(30) Priorität: 27.09.2019 DE 102019126189
(43) Veröffentlichungstag der Anmeldung: 31.03.2021
(73) Patentinhaber: B. Braun Avitum AG, 34212 Melsungen (DE)
(72) Erfinder: BOCZ, Máté, 1094 Budapest (HU); GOLARITS, István, 1073 Budapest (HU); TÉNYI, Botond, 1037 Budapest (HU)
(74) Vertreter: Winter, Brandl - Partnerschaft mbB

(56) Entgegenhaltungen:
- DE-T5-112014 001 324
- US-A1- 2019 001 046

## Beschreibung

Die vorliegende Offenbarung betrifft eine Blutbehandlungsvorrichtung zur Verwendung in (kontinuierlichen) Blutbehandlungs-/ Dialysetherapien, mit: einem extrakorporalen Blutkreislauf, einem Dialysator und einem Dialysierflüssigkeitskreislauf, wobei der extrakorporale Blutkreislauf und der Dialysierflüssigkeitskreislauf über eine in dem Dialysator vorgesehene (semipermeable) Membran, über welche Blut (unter Verwendung einer Dialysierflüssigkeit) gefiltert werden kann, voneinander getrennt sind, und der extrakorporale Blutkreislauf einen arteriellen Abschnitt stromaufwärts des Dialysators und einen venösen Abschnitt stromabwärts des Dialysators aufweist; einem Luftdetektor, welcher in dem venösen Abschnitt angeordnet ist und eingerichtet ist, den venösen Abschnitt ständig/ dauerhaft kontinuierlich dahingehend zu überwachen, ob sich Luft in dem venösen Abschnitt befindet; einer Vielzahl von Pumpen, darunter zumindest eine Blutpumpe, welche eine Pumpe in dem arteriellen Abschnitt ist und eingerichtet ist, Blut durch den extrakorporalen Blutkreislauf zu pumpen; einer venösen Schlauchklemme, welche eine Schlauchklemme in dem venösen Abschnitt ist und eingerichtet ist, den venösen Abschnitt selektiv abzuklemmen oder freizugeben; einer Benutzeroberfläche umfassend ein Display mit Touch-Screen; und einer Steuereinheit, welche eingerichtet ist, wenn sie von dem Luftdetektor eine Information erhält, dass sich Luft in dem venösen Abschnitt befindet, die Blutpumpe zu stoppen, die venöse Schlauchklemme zu schließen, einen Alarm auszugeben und auf dem Display ein Venöse-Luft-Entfernungsfenster anzuzeigen, welches Anweisungen für einen Anwender zur Entfernung der Luft in dem venösen Abschnitt und Statusberichte über die Entfernung der Luft anzeigt, und eine automatische Entfernung der Luft aus dem venösen Abschnitt anwenderinitiiert durchzuführen.

### Stand der Technik

Aus dem Stand der Technik sind bereits Blutbehandlungsvorrichtungen bekannt. Beispielsweise offenbart die WO 93/01858 A eine Blutbehandlungsvorrichtung mit einer Steuereinheit. Wenn Luft in einem venösen Abschnitt des extrakorporalen Blutkreislaufs erfasst wird, gibt die Steuereinheit einen Alarm aus und schließt automatisch eine venöse Schlauchklemme. Die Luft wird aus dem venösen Abschnitt entfernt, indem eine sterile Spritze in eine Entlüftungsleitung eines Filters einer Luftauffangkammer eingeführt wird, ein Ventil in der Entlüftungsleitung geöffnet wird und die Luft abgesaugt wird.

Auch offenbart ein weiteres Dokument, die US 9,795,731 B2, eine Blutbehandlungsvorrichtung mit einer Steuereinheit. Gemäß diesem Dokument wird Luft aus einem venösen Abschnitt eines extrakorporalen Blutkreislaufs mittels einer Blutpumpe entfernt.

Die US 2019/001046 A1 offenbart eine Blutbehandlungsvorrichtung gemäß der Präambel des Anspruchs 1. Eine weitere gattungsgemäße Blutbehandlungsvorrichtung ist aus der DE 11 2014 001324 T5 bekannt, wobei in diesem Dokument keine Anzeige von Statusberichten über die Entfernung der Luft auf dem Display vorgesehen ist.

Wenn sich Luft in dem venösen Abschnitt eines extrakorporalen Blutkreislaufs befindet, stellt dies grundsätzlich eine gefährliche Situation für einen Patienten dar. Aus dem Stand der Technik ist es bekannt, dass Luft, welche sich in dem venösen Abschnitt des extrakorporalen Blutkreislaufs befindet, entfernt werden muss und erst nach der Entfernung eine Blutbehandlungstherapie fortzusetzen ist. Der Stand der Technik hat jedoch grundsätzlich den Nachteil, dass die Luftentfernung aus dem venösen Abschnitt relativ kompliziert und umständlich und somit nicht anwenderfreundlich ist.

### Kurzbeschreibung der Offenbarung

Es ist also Aufgabe der vorliegenden Offenbarung, die Nachteile aus dem Stand der Technik zu vermeiden oder wenigstens zu mildern. Insbesondere soll die Blutbehandlungsvorrichtung so eingerichtet sein, dass Luft aus einem venösen Abschnitt des extrakorporalen Blutkreislaufs in einfacher Weise sowie anwenderfreundlich entfernt werden kann.

Diese Aufgabe wird bei einer gattungsgemäßen Blutbehandlungsvorrichtung dadurch gelöst, dass in dem venösen Abschnitt zusätzlich zu der venösen Schlauchklemme eine manuell betätigbare Klemme vorgesehen ist, mittels welcher ein Anwender selektiv den venösen Abschnitt abklemmen oder freigeben kann.

Die Vereinfachung und Anwenderfreundlichkeit wird gemäß der vorliegenden Offenbarung insbesondere durch die offenbarungsgemäße Kombination von automatisch von der Steuereinheit durchgeführten Schritten und manuell durch einen Anwender durchzuführenden Schritten erreicht. Offenbarungsgemäß veranlasst die Steuereinheit automatisch Schritte, welche durchgeführt werden, wenn sich Luft in dem venösen Abschnitt befindet. Die Steuereinheit stoppt offenbarungsgemäß die Blutpumpe, schließt die venöse Schlauchklemme, gibt den Alarm aus und zeigt das Venöse-Luft-Entfernungsfenster auf dem Display an. Die spezielle Anzeige auf dem Display, welche dem Anwender die Schritte aufzeigt, die er zur Entfernung der Luft aus dem venösen Abschnitt durchführen muss, wird vorliegend als Venöse-Luft-Entfernungsfenster bezeichnet. Das Venöse-Luft-Entfernungsfenster nimmt den Anwender an der Hand und erläutert ihm die manuell durchzuführenden Schritte und gibt ihm nach entsprechender Durchführung der manuell durchzuführenden Schritte/ eines manuell durchzuführenden Schritts entsprechendes Feedback in Form von Statusberichten/ eines Statusberichts. Die offenbarungsgemäß durchzuführenden manuellen Schritte zeichnen sich durch ihre Einfachheit aus, so dass ein Anwender diese ohne vorheriges Training/ ohne vorherige Einführung durchführen kann. Schließlich ist die Steuereinheit eingerichtet, die automatische Entfernung der Luft aus dem venösen Abschnitt durchzuführen, wenn ein Anwender dies initiiert, beispielsweise durch eine entsprechende Eingabe auf der Benutzeroberfläche.

Vorteilhafte Ausführungsformen sind in den Unteransprüchen beansprucht und werden nachfolgend erläutert.

Es ist vorteilhaft, wenn die manuell betätigbare Klemme in dem venösen Abschnitt unmittelbar nach dem Dialysator/ stromabwärts des Dialysators (d.h. an dem Dialysatorausgang in dem venösen Abschnitt) und somit stromaufwärts der venösen Schlauchklemme angeordnet vorgesehen ist.

Die Steuereinheit ist in vorteilhafter Weise eingerichtet, wenn sich Luft in dem venösen Abschnitt befindet, in dem Venöse-Luft-Entfernungsfenster eine Anweisung für den Anwender anzuzeigen, dass er die manuell betätigbare Klemme in dem venösen Abschnitt schließen soll.

Die vorliegende Offenbarung zeichnet sich neben der offenbarungsgemäßen Kombination aus automatisch durchgeführten Schritten und manuell durchzuführenden Schritten insbesondere durch das beanspruchte Vorsehen der manuell betätigbaren Klemme zusätzlich zu der venösen Schlauchklemme im venösen Abschnitt des extrakorporalen Blutkreislaufs aus. Insbesondere ermöglicht es die manuell betätigbare Klemme, dass die manuell vorzunehmenden Schritte sehr einfach und anwenderfreundlich durchführbar sind.

Die Steuereinheit ist bevorzugt eingerichtet, wenn sich Luft in dem venösen Abschnitt befindet, in dem Venöse-Luft-Entfernungsfenster einen Luftentfernungs-Button anzuzeigen, welcher durch den Anwender durch Drücken auf den Touch-Screen betätigbar ist. Der Luftentfernungs-Button ist gegebenenfalls (falls erforderlich) auch mehrmals bzw. wiederholt betätigbar, beispielsweise wenn sich nach einer Durchführung des Luftentfernungsprozesses immer noch Luft in dem venösen Abschnitt befindet. Durch ein Drücken des Luftentfernungs-Buttons von Seiten des Anwenders wird somit offenbarungsgemäß die automatische Luftentfernung aktiviert.

Die Steuereinheit ist bevorzugt eingerichtet, wenn sie erfasst, dass der Luftentfernungs-Button von dem Anwender gedrückt wurde, einen Unterdruck in dem venösen Abschnitt einzustellen und die venöse Schlauchklemme zu öffnen, um die Luft aus dem venösen Abschnitt herauszusaugen.

In vorteilhafter Weise ist die Steuereinheit eingerichtet, wenn sie erfasst, dass der Luftentfernungs-Button von dem Anwender gedrückt wurde, zu überprüfen, ob die manuell betätigbare Klemme geschlossen ist, indem sie eine Druckänderung in dem extrakorporalen Kreislauf überwacht, und einen Alarm zum Schließen der manuell betätigbaren Klemme zu erzeugen, wenn die manuell betätigbare Klemme nicht geschlossen ist. Ein Alarm zum Schließen der manuell betätigbaren Klemme ist gemäß der vorliegenden Offenbarung ein Alarm, welcher einen Anwender darauf hinweist (optisch und/oder akustisch), dass er die manuell betätigbare Klemme schließen sollte.

Bevorzugt ist die Steuereinheit eingerichtet, wenn sie erfasst, dass der Luftentfernungs-Button von dem Anwender gedrückt wurde, einen Unterdruck, welcher geringer als ein vordefinierte Wert ist, in einer in dem venösen Abschnitt angeordneten venösen Luftfalle durch Betätigen/ Aktivieren einer Pegel-/ bzw. Levelregulierungspumpe zu erzeugen, und im Anschluss die venöse Schlauchklemme zu öffnen, zum automatischen Heraussagen der Luft aus dem venösen Abschnitt. Dabei ist der vordefinierte Wert bevorzugt -50 mmHg.

Vorzugsweise ist die Steuereinheit eingerichtet, wenn sie erfasst, dass die Luft aus dem venösen Abschnitt herausgesaugt wurde, die venöse Schlauchklemme wieder zu schließen und in dem Venöse-Luft-Entfernungsfenster anzuzeigen, dass die Entfernung der Luft abgeschlossen ist.

Ferner ist es von Vorteil, wenn die Steuereinheit eingerichtet ist, wenn sie erfasst, dass die Entfernung der Luft abgeschlossen ist, in dem Venöse-Luft-Entfernungsfenster eine Aufforderung an den Anwender anzuzeigen, dass er die manuell betätigbare Klemme wieder öffnen soll.

Bevorzugt ist die Steuereinheit eingerichtet, eine Therapie wieder aufzunehmen, wenn der Anwender auf dem Touch-Screen bestätigt hat, dass er die manuell betätigbare Klemme geöffnet hat, und die Steuereinheit von dem Luftdetektor die Information erhält, dass sich keine Luft mehr in dem venösen Abschnitt befindet.

Weiterhin ist es von Vorteil, wenn die Steuereinheit eingerichtet ist, zu überprüfen, ob die manuell betätigbare Klemme nach einer Wiederaufnahme der (Blutbehandlungs-) Therapie tatsächlich geöffnet ist, indem eine Druckänderung in dem extrakorporalen Kreislauf überwacht wird, und falls die manuell betätigbare Klemme noch geschlossen ist, einen Alarm zum Öffnen der manuell betätigbaren Klemme zu erzeugen. Ein Alarm zum Öffnen der manuell betätigbaren Klemme ist gemäß der vorliegenden Offenbarung ein Alarm, welcher einen Anwender darauf hinweist (optisch und/oder akustisch), dass er die manuell betätigbare Klemme öffnen sollte.

Es ist zweckmäßig, wenn der extrakorporale Blutkreislauf und der Dialysierflüssigkeitskreislauf als Einwegschläuche ausgebildet sind, welche an einer an der Dialysemaschine vorgesehenen Schnittstelle aufgenommen sind.

Bevorzugt weist die Blutbehandlungsvorrichtung eine Wägevorrichtung, insbesondere Wägezelle, zum Messen des Gewichts eines Beutels, insbesondere Einwegbeutels, welcher ein für die Blutbehandlung erforderliches Fluid enthält, auf.

Bevorzugt umfasst die Vielzahl von Pumpen neben der Blutpumpe zumindest eine Substitutionslösungspumpe, eine Spritzenpumpe und eine Ultrafiltratpumpe.

Die Blutbehandlungsvorrichtung weist ferner vorzugsweise ein Bar-Code-Lesegerät auf, welches eingerichtet ist, auf Einwegartikel wie etwa Einwegschläuche oder auf deren Umverpackung aufgebrachte Bar-Codes zu lesen.

Die Blutbehandlungsvorrichtung ist bevorzugt zur drahtgebundenen Kommunikation eingerichtet.

Die Steuereinheit der Blutbehandlungsvorrichtung ist vorzugsweise als zumindest ein Prozessor, vorzugsweise mehrere Prozessoren, ausgebildet.

Mit anderen Worten betrifft die Offenbarung eine Dialysemaschine. Die Dialysemaschine enthält ein Bar-Code-Lesegerät. Weiterhin enthält die Dialysemaschine eine Benutzeroberfläche bzw. ein Display mit Touch-Screen. Die Dialysemaschine weist ferner eine Schnittstelle/ Interface für ein Einwegschlauchset auf, welches eine Blutseite und eine Dialysierflüssigkeitsseite enthält, die voneinander durch eine (semi-) permeable/ durchlässige Membran getrennt sind, um Blut (unter Verwendung einer Dialysierflüssigkeitslösung/ Dialyselösung) zu filtern. Eine Substitutionslösung/ Ersatzlösung wird der Blutseite vor/nach einem Dialysator zugeführt. Die Dialysemaschine weist eine Blutpumpe, eine Spritzenpumpe, eine Ultrafiltratpumpe, eine Substitutionslösungspumpe etc. auf. Die Dialysemaschine ist zur drahtgebundenen Kommunikation eingerichtet/ weist verdrahtete bzw. drahtgebundene Kommunikationseinrichtungen auf. Die Dialysemaschine zeichnet sich durch eine Software aus, welche besonders für eine Verwendung in kontinuierlichen Dialysetherapien, beispielsweise Nierenersatztherapien geeignet ist. Die Software läuft dabei auf einer Vielzahl von Prozessoren innerhalb der Dialysemaschine. Weiterhin weist die Dialysemaschine eine Energieverwaltungseinrichtung (integrierter Schaltkreis) auf. Die Dialysemaschine enthält darüber hinaus Wägevorrichtungen, insbesondere Wägezellen, welche das Gewicht von Einwegbeuteln messen, welche die für die Dialysetherapie erforderlichen Fluide (z.B. Dialysierflüssigkeitslösung, Substitutionslösung) enthalten.

Die Steuereinheit der vorliegenden Offenbarung stellt eine automatische Entfernung von Luft bereit. Wenn sich Luft in einem venösen Abschnitt eines extrakorporalen Blutkreislaufs befindet, stellt dies grundsätzlich eine gefährliche Situation für einen Patienten dar. Die offenbarungsgemäße Dialysemaschine (insbesondere die Steuereinheit derselben) überwacht kontinuierlich/ dauerhaft den venösen Abschnitt des extrakorporalen Blutkreislaufs mit einem (Sicherheits-) Luftdetektor in dem venösen Abschnitt. Wenn der "Luft in dem venösen Abschnitt"-Alarm auftritt, erscheint das Venöse-Luft-Entfernungsfenster auf der Benutzeroberfläche.

Wenn Luft in dem venösen Abschnitt erfasst wird, stoppt die Dialysemaschine die Blutpumpe, schließt die venöse Schlauchklemme, erzeugt einen entsprechenden Alarm und zeigt das "Luftentfernung aus venösem Abschnitt"-Fenster an. Ein Anwender wird auf dem Fenster aufgefordert, eine manuell betätigbare Klemme an dem venösen Abschnitt zu schließen. Diese Klemme weist bevorzugt eine spezifische Farbe auf, beispielsweise blau, und der Anwender wird dementsprechend aufgefordert, die Klemme mit der spezifischen Farbe, beispielsweise die blaue Klemme, zu schließen. Auf dem "Luftentfernung aus venösem Abschnitt"-Fenster wird der Anwender weiterhin aufgefordert einen Luftentfernungs-Button zu drücken. Wenn der Luftentfernungs-Button von dem Anwender gedrückt wird, verringert die Dialysemaschine (die Steuereinheit derselben) den Druck in dem venösen Abschnitt auf einen Unterdruck von bevorzugt - 50 mmHg, indem sie eine Luftpumpe (eine Niveau-! Levelregulierungspumpe) ansteuert. Diese Luftpumpe ist im Übrigen bevorzugt an einen Verteiler angeschlossen und es wird über eine Vielzahl von Ventilen gesteuert, an welchem Punkt des Einwegschlauchsets die Luftpumpe Luft hinzufügt bzw. von welchem Punkt des Einwegschlauchsets die Luftpumpe Luft entfernt, um den Druck in dem venösen Abschnitt entsprechend einzustellen/ zu verringern. Danach öffnet die Dialysemaschine (die Steuereinheit derselben) die venöse Schlauchklemme und saugt die Luft heraus. Dann schließt die Dialysemaschine die venöse Schlauchklemme wieder. Im Anschluss kann der Anwender den venösen Abschnitt untersuchen. Falls notwendig kann er den Luftentfernungs-Button erneut drücken. Wenn die Luft entfernt ist, öffnet der Anwender die manuelle betätigbare (blaue) Klemme an dem venösen Abschnitt und drückt einen OK-Button, um die Blutbehandlungstherapie wiederaufzunehmen. Die Dialysemaschine nimmt die Therapie wieder auf, wenn keine Luft mehr in dem venösen Abschnitt erfasst wird.

### Kurzbeschreibung der Figuren

Die Offenbarung wird nachfolgend mit Hilfe von Figuren weiter erläutert. Es zeigen:
- Fig. 1: eine schematische Ansicht einer Blutbehandlungsvorrichtung gemäß der vorliegenden Offenbarung; und
- Fig. 2: ein Venöse-Luft-Entfernungsfenster gemäß der vorliegenden Offenbarung.

### Figurenbeschreibung

Die Figuren sind lediglich schematischer Natur und dienen ausschließlich dem Verständnis der vorliegenden Offenbarung. Gleiche Elemente sind dabei mit denselben Bezugszeichen versehen.

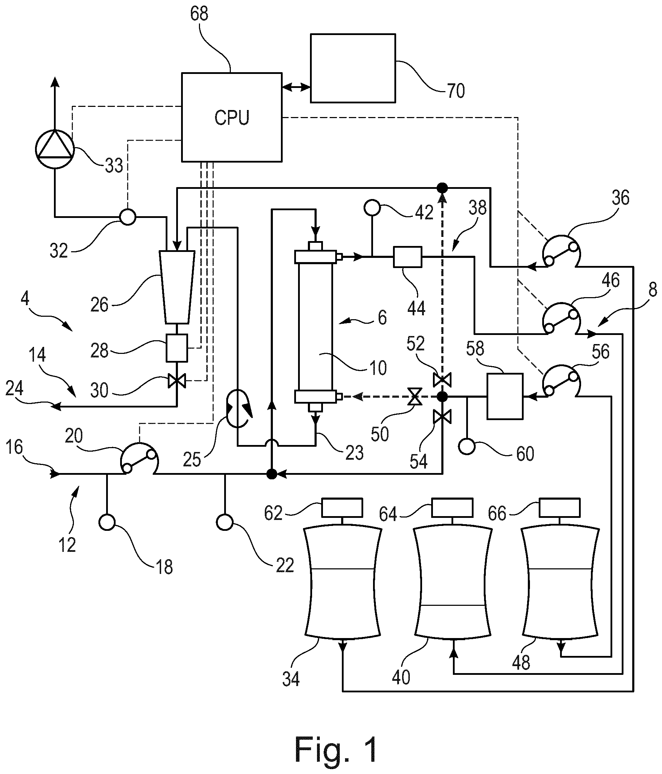

Fig. 1 zeigt eine schematische Ansicht einer extrakorporalen Blutbehandlungsvorrichtung (Dialysemaschine) 2. Die Blutbehandlungsvorrichtung 2 ist grundsätzlich eingerichtet, sowohl in kontinuierlichen als auch in intermittierenden Blutbehandlungstherapien, insbesondere Nierenersatztherapien, verwendet zu werden. Die Blutbehandlungsvorrichtung 2 ist insbesondere als eine Akutdialysemaschine bzw. als ein Akut-Dialysegerät ausgebildet und ist somit im Wesentlichen zur Verwendung auf Intensivstationen bei überwiegend instabilen Patienten vorbereitet. Mit der Blutbehandlungsvorrichtung 2 der vorliegenden Offenbarung können grundsätzlich eine Vielzahl von verschiedenen Blutbehandlungstherapien (z.B. langsame kontinuierliche Ultrafiltration (SCUF), kontinuierliche venös-venöse Hämofiltration (CWH), kontinuierliche venös-venöse Hämodialyse (CWHD), kontinuierliche venös-venöse Hämodiafiltration (CWHDF), therapeutischer Plasmaaustauch (TPE), etc.), Dilutionsmodi (z.B. Prädilution, Postdilution, Prä- und Postdilution), sowie Antikoagulationsarten (z.B. keine, Heparin, Citrat, etc.) durchgeführt werden.

Die Blutbehandlungsvorrichtung 2 weist grundsätzlich einen extrakorporalen Kreislauf 4, einen Dialysator (Hämofilter) 6 und einen Dialysierflüssigkeitskreislauf 8 auf. Der extrakorporale Kreislauf 4 und der Dialysierflüssigkeitskreislauf 8 sind über eine in dem Dialysator 6 vorgesehene Membran 10, über welche Blut unter Verwendung einer Dialysierflüssigkeitslösung oder ohne Verwendung einer Dialysierflüssigkeitslösung gefiltert werden kann, voneinander getrennt.

Der extrakorporale Kreislauf 4 umfasst einen arteriellen Abschnitt 12 und einen venösen Abschnitt 14. Dabei ist grundsätzlich vorgesehen, dass der arterielle Abschnitt 12, insbesondere ein Ende desselben, an eine Arterie eines Patienten, insbesondere eines Intensivpatienten, angeschlossen bzw. mit derselben verbunden werden soll. Weiterhin ist vorgesehen, dass der venöse Abschnitt 14, insbesondere ein Ende desselben, an eine Vene eines Patienten, insbesondere eines Intensivpatienten, angeschlossen bzw. mit derselben verbunden werden soll.

Der arterielle Abschnitt 12 weist ausgehend von einem arteriellen Ende 16 in einer Blut-Strömungsrichtung hin zu dem Dialysator 6 einen arteriellen Drucksensor 18, eine (arterielle) Blutpumpe 20 und einen Dialysatoreingangsdrucksensor 22 auf. Der venöse Abschnitt 14 weist ausgehend von dem Dialysator 6 (einem Dialysatorausgang 23) in einer Blut-Strömungsrichtung hin zu einem venösen Ende 24 eine manuell betätigbare Klemme 25, eine venöse Expansionskammer bzw. Luftfalle 26, einen Sicherheitsluftdetektor/ Luftdetektor 28 und eine venöse Schlauchklemme 30 auf. Mittels der manuell betätigbaren Klemme 25 kann ein Anwender selektiv den venösen Abschnitt 14 abklemmen oder freigeben. An/ hinter der venösen Expansionskammer 26 kann ein venöser Druck über einen venösen Drucksensor 32 gemessen werden. Eine hinter dem venösen Drucksensor 32 vorgesehene Level-/ bzw. Pegelregulierungspumpe 33 kann Luft in die venöse Expansionskammer bzw. Luftfalle 26 drücken oder Luft aus dieser herausziehen und ist somit grundsätzlich eingerichtet und vorgesehen, einen Unterdruck oder einen Überdruck in der venösen Expansionskammer bzw. Luftfalle 26 und somit in dem venösen Abschnitt 14 zu erzeugen.

Wie aus Fig. 1 hervorgeht, ist die venöse Expansionskammer 26 mit einem Substitutionslösungsbeutel/ -behälter 34 verbunden. Eine Substitutionslösungspumpe 36 ist dabei vorgesehen und eingerichtet, eine Substitutionslösung aus dem Substitutionslösungsbeutel 34 in den extrakorporalen Blutkreislauf 4, insbesondere in den venösen Abschnitt 14 desselben (in die venöse Expansionskammer 26) zu pumpen.

Der Dialysierflüssigkeitskreislauf 8 weist zumindest einen Abfluss 38 für Ultrafiltrat/ verbrauchte Dialysierflüssigkeit (Dialysat)/ eine sonstige Flüssigkeit auf. Grundsätzlich kann das Ultrafiltrat/ Dialysat/ die sonstige Flüssigkeit über den Abfluss 38 von dem Dialysator 6 hin zu einem Sammelbeutel/ -behälter 40 für Ultrafiltrat/ Dialysat/ etc. fließen. In dem Abfluss 38 sind ausgehend von dem Dialysator 6 in einer Strömungsrichtung hin zu dem Sammelbeutel 40 ein Ultrafiltratdrucksensor 42, ein Blutleckdetektor 44 und eine Ultrafiltratpumpe 46 angeordnet bzw. vorgesehen.

Wie aus Fig. 1 weiter hervorgeht, ist neben dem Substitutionslösungsbeutel 34 und dem Sammelbeutel 40 noch ein weiterer Beutel/ Behälter 48 vorgesehen. Der Beutel 48 kann in Abhängigkeit von der gewünschten durchzuführenden Blutbehandlungstherapie beispielsweise eine Substitutionslösung /-flüssigkeit oder eine Dialysierflüssigkeit enthalten.

Wenn beispielsweise mit der extrakorporalen Blutbehandlungsvorrichtung 2 eine Hämodialyse-/ eine Hämodiafiltrationsbehandlung etc. durchgeführt werden soll, also eine Blutbehandlungstherapie, in welcher Dialysierflüssigkeit durch den Dialysator 6 strömt und somit ein Stofftransport von dem extrakorporalen Kreislauf 4 zu dem Dialysierflüssigkeitskreislauf 8 sowohl über Diffusion als auch über Konvektion erfolgt, enthält der Beutel 48 Dialysierflüssigkeit. Wird nun ein erstes Ventil 50 geöffnet und werden sowohl ein zweites Ventil 52 als auch ein drittes Ventil 54 geschlossen, kann die Dialysierflüssigkeit über eine Pumpe 56 zum Dialysator 6 gepumpt werden.

Soll alternativ mit der extrakorporalen Blutbehandlungsvorrichtung 2 beispielsweise eine Hämofiltration etc. durchgeführt werden, also eine Blutbehandlungstherapie, in welcher keine Dialysierflüssigkeit durch den Dialysator 6 strömt und somit ein Stofftransport von dem extrakorporalen Kreislauf 4 zu dem Dialysierflüssigkeitskreislauf 8 lediglich über Konvektion/ Filtration erfolgt, kann der Beutel 48 eine Substitutionslösung enthalten. Werden nun das erste Ventil 50 und das zweite Ventil 52 geschlossen und wird das dritte Ventil 54 geöffnet, kann die Substitutionslösung aus dem Beuel 48 in den arteriellen Abschnitt 12 des extrakorporalen Kreislaufs 4 gepumpt werden (Prädilution). Werden das erste Ventil 50 und das dritte Ventil 54 geschlossen und wird das zweite Ventil 52 geöffnet, kann die Substitutionslösung aus dem Beuel 48 in den venösen Abschnitt 14 des extrakorporalen Kreislaufs 4 gepumpt werden (Postdilution). Wird das erste Ventil 50 geschlossen und werden das zweite Ventil 52 und das dritte Ventil 54 geöffnet, kann die Substitutionslösung aus dem Beutel 48 sowohl in den arteriellen Abschnitt 12 als auch in den venösen Abschnitt 14 des extrakorporalen Kreislaufs gepumpt werden (Prä- und Postdilution). Eine Prä- und Postdilution kann gemäß der vorliegenden Offenbarung auch dadurch erreicht werden, dass die Substitutionslösung aus dem Substitutionslösungsbeutel 34 mittels der Substitutionslösungspumpe 36 in den venösen Abschnitt 14 des extrakorporalen Kreislaufs 4 gepumpt wird (Postdilution), und dass gleichzeitig die Substitutionslösung aus dem Beutel 48 mittels der Pumpe (Substitutionslösungspumpe) 56 in den arteriellen Abschnitt 12 des extrakorporalen Kreislaufs 4 gepumpt wird (Prädilution).

Wie aus Fig. 1 weiter hervorgeht, sind zwischen der Pumpe 56 und der Ventilanordnung bestehend aus dem ersten Ventil 50, dem zweiten Ventil 52 und dem dritten Ventil 54 ein Flüssigkeitswärmer 58 und ein Drucksensor 60 vorgesehen.

An den drei Beuteln, also dem Substitutionslösungsbeutel 34, dem Sammelbeutel 40 und dem Beutel 48, sind jeweils Wägezellen, nämlich eine erste Wägezelle 62, eine zweite Wägezelle 64 und eine dritte Wägezelle 66 angeordnet. Die erste Wägezelle 62 ist dabei grundsätzlich eingerichtet, das Gewicht des Substitutionslösungsbeutels 34 zu messen bzw. zu überwachen. Die zweite Wägezelle 64 ist grundsätzlich eingerichtet, das Gewicht des Sammelbeutels 40 zu messen bzw. zu überwachen. Die dritte Wägezelle 66 ist grundsätzlich eingerichtet, das Gewicht des Beutels 48 zu messen bzw. zu überwachen.

Die extrakorporale Blutbehandlungsvorrichtung 2 weist weiterhin eine Steuereinheit (CPU) 68 auf, welche Informationen von den in der Blutbehandlungsvorrichtung 2 vorgesehenen Sensoren erhält, und welche die in der Blutbehandlungsvorrichtung 2 vorgesehenen Aktoren ansteuert. Offenbarungsgemäß wird somit insbesondere eine softwareunterstützte Therapie bereitgestellt. Die Steuereinheit 68 erhält dabei insbesondere Informationen von dem arteriellen Drucksensor 18, dem Dialysatoreingangsdrucksensor 22, dem Sicherheitsluftdetektor 28, dem venösen Drucksensor 32, dem Ultrafiltratdrucksensor 42, dem Blutleckdetektor 44, dem Drucksensor 60, der ersten Wägezelle 62, der zweiten Wägezelle 64, der dritten Wägezelle 66, etc. Die Steuereinheit 68 steuert insbesondere die Blutpumpe 20, die venöse Schlauchklemme 30, die Level-/ bzw. Pegelregulierungspumpe 33, die Substitutionslösungspumpe 36, die Ultrafiltratpumpe 46, das erste Ventil 50, das zweite Ventil 52, das dritte Ventil 54, die Pumpe 56, den Flüssigkeitswärmer 58, etc. an. Weiterhin ist die Steuereinheit 68 im Austausch mit einer als Display mit Touch-Screen ausgebildeten Benutzeroberfläche 70. Beispielsweise kann die Steuereinheit 68 eingerichtet sein, Informationen auf der Benutzeroberfläche 70 anzuzeigen. Weiterhin können Eingaben von einem Nutzer/ Anwender auf der Benutzeroberfläche 70 an die Steuereinheit 68 weitergegeben werden.

Die Steuereinheit 68 der vorliegenden Offenbarung erhält grundsätzlich Informationen von dem Sicherheitsluftdetektor 28, welcher in dem venösen Abschnitt 14 eines extrakorporalen (Blut-) Kreislaufs 4 angeordnet ist. Wenn Luft von dem Sicherheitsluftdetektor 28 erfasst wird, stoppt die Steuereinheit 68 die (arterielle) Blutpumpe 20 (bevorzugt alle Pumpen, also neben der arteriellen Blutpumpe 20 auch die Substitutionslösungspumpe 36, die Ultrafiltratpumpe 46 und die Pumpe 56), schließt die venöse Schlauchklemme 30, erzeugt einen Alarm und zeigt auf einem Display 72 der Benutzeroberfläche 70 ein Venöse-Luft-Entfernungsfenster 74 an.

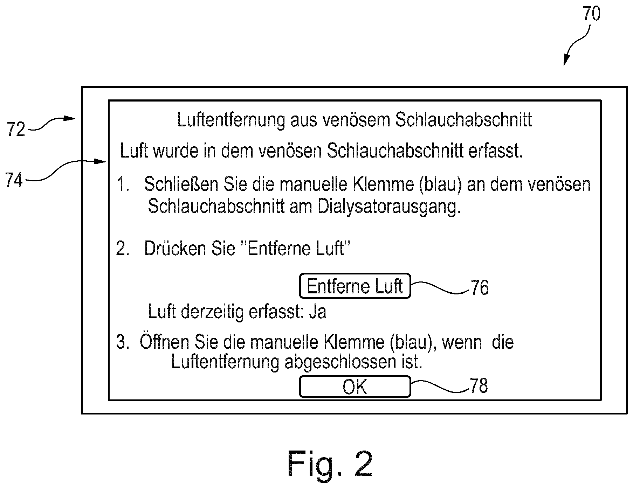

Fig. 2 zeigt das Venöse-Luft-Entfernungsfenster 74. Auf dem Fenster wird ein Anwender darauf hingewiesen, dass Luft in dem venösen Abschnitt 14 des extrakorporalen Kreislaufs 4 erfasst/ detektiert wurde. Der Anwender wird aufgefordert, in einem ersten Schritt eine manuell betätigbare (blaue) Klemme 25 an dem venösen Abschnitt 14 zu schließen, welche sich nahe/ an dem Dialysatorausgang 23 des Dialysators 6 der extrakorporalen Blutbehandlungsvorrichtung 2 befindet.

In einem zweiten Schritt wird der Anwender auf dem Venöse-Luft-Entfernungsfenster 74 aufgefordert, einen Luftentfernungs-Button 76 zu drücken. Wenn der Luftentfernungs-Button 76 von dem Anwender gedrückt wird, überprüft die Steuereinheit 68, ob die manuell betätigbare Klemme 25 tatsächlich geschlossen ist, indem sie eine Druckänderung in dem extrakorporalen Kreislauf 4, insbesondere in dem venösen Abschnitt 14 desselben, mittels dem venösen Drucksensor 32 überwacht. Wenn die Steuereinheit 68 feststellt, dass die manuell betätigbare Klemme 25 nicht geschlossen ist, erzeugt die Steuereinheit 68 einen (akustischen und/oder optischen) Alarm, welcher dem Anwender mitteilt, dass er die manuell betätigbare Klemme 25 schließen muss.

Wenn die Steuereinheit 68 feststellt, dass die manuell betätigbare Klemme 25 geschlossen ist, verringert die Steuereinheit 68 der Blutbehandlungsvorrichtung 2 den Druck in dem venösen Abschnitt 14, insbesondere in der venösen Expansionskammer/ Luftfalle 26, auf einen Unterdruck/ negativen Druck, welcher geringer als ein vordefinierter Wert (bevorzugt - 50 mmHg) ist, indem sie die Niveau-/ Level/- Pegelregulierungspumpe 33 ansteuert.

Danach öffnet die Steuereinheit 68 der Blutbehandlungsvorrichtung 2 die venöse Schlauchklemme 30, wodurch die Luft automatisch herausgesaugt wird.

Wenn die Steuereinheit 68 erfasst, dass die Luft aus dem venösen Abschnitt 14 herausgesaugt wurde, schließt die Steuereinheit 68 die venöse Schlauchklemme 30 wieder. Im Anschluss kann der Anwender den venösen Abschnitt 14 untersuchen. Falls notwendig kann er den Luftentfernungs-Button 76 erneut drücken. Wenn die Luft entfernt ist, zeigt das Venöse-Luft-Entfernungsfenster 74 dies dem Anwender an und gibt ihm somit einen Statusbericht über die Entfernung der Luft. Der Anwender kann dann in einem dritten Schritt die manuell betätigbare (blaue) Klemme 25 an dem venösen Abschnitt 14 wieder öffnen und einen OK-Button 78 drücken, um die Blutbehandlungstherapie wiederaufzunehmen. Die Blutbehandlungsvorrichtung 2 nimmt die Therapie wieder auf, wenn keine Luft mehr in dem venösen Abschnitt 14 von dem Sicherheitsluftdetektor 28 erfasst wird.

Nach der Wiederaufnahme der Blutbehandlungstherapie überprüft die Steuereinheit 68, ob die manuell betätigbare Klemme 25 tatsächlich geöffnet ist. Dazu überwacht die Steuereinheit 68 eine von dem venösen Drucksensor 32 erfasste Druckänderung in dem venösen Abschnitt 14 des extrakorporalen Kreislaufs 4. Falls die manuell betätigbare Klemme 25 noch geschlossen ist, erzeugt die Steuereinheit 68 einen (akustischen und/oder optischen) Alarm, welcher dem Anwender anzeigt, dass er die manuell betätigbare Klemme 25 öffnen sollte.

### Bezugszeichenliste

- 2: Blutbehandlungsvorrichtung! Dialysemaschine
- 4: extrakorporaler Kreislauf
- 6: Dialysator
- 8: Dialysierflüssigkeitskreislauf
- 10: Membran
- 12: arterieller Abschnitt
- 14: venöser Abschnitt
- 16: arterielles Ende
- 18: arterieller Drucksensor
- 20: (arterielle) Blutpumpe
- 22: Dialysatoreingangsdrucksensor
- 23: Dialysatorausgang
- 24: venöses Ende
- 25: manuell betätigbare Klemme
- 26: venöse Expansionskammer/ Luftfalle
- 28: Sicherheitsluftdetektor
- 30: venöse Schlauchklemme
- 32: venöser Drucksensor
- 33: Level-/ bzw. Pegelregulierungspumpe
- 34: Substitutionslösungsbeutel
- 36: Substitutionslösungspumpe
- 38: Abfluss
- 40: Sammelbeutel
- 42: Ultrafiltratdrucksensor
- 44: Blutleckdetektor
- 46: Ultrafiltratpumpe
- 48: Beutel
- 50: erstes Ventil
- 52: zweites Ventil
- 54: drittes Ventil
- 56: Pumpe
- 58: Flüssigkeitswärmer
- 60: Drucksensor
- 62: erste Wägezelle
- 64: zweite Wägezelle
- 66: dritte Wägezelle
- 68: Steuereinheit
- 70: Benutzerschnittstelle
- 72: Display
- 74: Venöse-Luft-Entfernungsfenster
- 76: Luftentfernungs-Button
- 78: OK-Button

## Patentansprüche

1. Blutbehandlungsvorrichtung (2) zur Verwendung in Blutbehandlungstherapien mit:
einem extrakorporalen Blutkreislauf (4), einem Dialysator (6) und einem Dialysierflüssigkeitskreislauf (8), wobei der extrakorporale Blutkreislauf (4) und der Dialysierflüssigkeitskreislauf (8) über eine in dem Dialysator (6) vorgesehene Membran (10), über welche Blut gefiltert werden kann, voneinander getrennt sind, und der extrakorporale Blutkreislauf (4) einen arteriellen Abschnitt (12) stromaufwärts des Dialysators (6) und einen venösen Abschnitt (14) stromabwärts des Dialysators (6) aufweist;
einem Luftdetektor (28), welcher in dem venösen Abschnitt (14) angeordnet ist und eingerichtet ist, den venösen Abschnitt (14) kontinuierlich dahingehend zu überwachen, ob sich Luft in dem venösen Abschnitt (14) befindet;
einer Vielzahl von Pumpen (20, 36, 46, 56), darunter zumindest eine Blutpumpe (20), welche eine Pumpe in dem arteriellen Abschnitt (12) ist und eingerichtet ist, Blut durch den extrakorporalen Blutkreislauf (4) zu pumpen;
einer venösen Schlauchklemme (30), welche eine Schlauchklemme in dem venösen Abschnitt (14) ist und eingerichtet ist, den venösen Abschnitt (14) selektiv abzuklemmen oder freizugeben;
einer Benutzeroberfläche (70) umfassend ein Display (72) mit Touch-Screen; und
einer Steuereinheit (68), welche eingerichtet ist,
wenn sie von dem Luftdetektor (28) eine Information erhält, dass sich Luft in dem venösen Abschnitt (14) befindet, die Blutpumpe (20) zu stoppen, die venöse Schlauchklemme (30) zu schließen, einen Alarm auszugeben und auf dem Display (72) ein Venöse-Luft-Entfernungsfenster (74) anzuzeigen, welches Anweisungen für einen Anwender zur Entfernung der Luft in dem venösen Abschnitt (14) und Statusberichte über die Entfernung der Luft anzeigt; und
eine automatische Entfernung der Luft aus dem venösen Abschnitt (14) anwenderinitiiert durchzuführen,
**dadurch gekennzeichnet, dass**
in dem venösen Abschnitt (14) zusätzlich zu der venösen Schlauchklemme (30) eine manuell betätigbare Klemme (25) vorgesehen ist, mittels welcher ein Anwender selektiv den venösen Abschnitt (14) abklemmen oder freigeben kann.

2. Blutbehandlungsvorrichtung (2) nach Anspruch 1, **dadurch gekennzeichnet, dass** die manuell betätigbare Klemme (25) in dem venösen Abschnitt (14) an dem Dialysatorausgang (23) und somit stromaufwärts der venösen Schlauchklemme (30) angeordnet ist.

3. Blutbehandlungsvorrichtung (2) nach Anspruch 1 oder 2, **dadurch gekennzeichnet, dass** die Steuereinheit (68) eingerichtet ist, wenn sich Luft in dem venösen Abschnitt (14) befindet, in dem Venöse-Luft-Entfernungsfenster (74) eine Anweisung für den Anwender anzuzeigen, dass er die manuell betätigbare Klemme (25) in dem venösen Abschnitt (14) schließen soll.

4. Blutbehandlungsvorrichtung (2) nach Anspruch 3, **dadurch gekennzeichnet, dass** die Steuereinheit (68) eingerichtet ist, wenn sich Luft in dem venösen Abschnitt (14) befindet, in dem Venöse-Luft-Entfernungsfenster (74) einen Luftentfernungs-Button (76) anzuzeigen, welcher durch den Anwender durch Drücken auf den Touch-Screen betätigbar ist.

5. Blutbehandlungsvorrichtung (2) nach Anspruch 4, **dadurch gekennzeichnet, dass** die Steuereinheit (68) eingerichtet ist, wenn sie erfasst, dass der Luftentfernungs-Button (76) von dem Anwender gedrückt wurde, zu überprüfen, ob die manuell betätigbare Klemme (25) geschlossen ist, indem sie eine Druckänderung in dem extrakorporalen Kreislauf (4) überwacht, und einen Alarm zum Schließen der manuell betätigbaren Klemme (25) zu erzeugen, wenn die manuell betätigbare Klemme (25) nicht geschlossen ist.

6. Blutbehandlungsvorrichtung (2) nach Anspruch 4 oder 5, **dadurch gekennzeichnet, dass** die Steuereinheit (68) eingerichtet ist, wenn sie erfasst, dass der Luftentfernungs-Button (76) von dem Anwender gedrückt wurde, einen Unterdruck, welcher geringer als ein vordefinierter Wert ist, in einer in dem venösen Abschnitt (14) angeordneten venösen Luftfalle (26) durch Betätigen/ Aktivieren einer Pegel-/ bzw. Levelregulierungspumpe (33) zu erzeugen, und im Anschluss die venöse Schlauchklemme (30) zu öffnen, zum automatischen Heraussagen der Luft aus dem venösen Abschnitt (14).

7. Blutbehandlungsvorrichtung (2) nach Anspruch 6, **dadurch gekennzeichnet, dass** der vordefinierte Wert -50 mmHg ist.

8. Blutbehandlungsvorrichtung (2) nach Anspruch 6 oder 7, **dadurch gekennzeichnet, dass** die Steuereinheit (68) eingerichtet ist, wenn sie erfasst, dass die Luft aus dem venösen Abschnitt (14) herausgesaugt wurde, die venöse Schlauchklemme (30) wieder zu schließen und in dem Venöse-Luft-Entfernungsfenster (74) anzuzeigen, dass die Entfernung der Luft abgeschlossen ist.

9. Blutbehandlungsvorrichtung (2) nach Anspruch 8, **dadurch gekennzeichnet, dass** die Steuereinheit (68) eingerichtet ist, wenn sie erfasst, dass die Entfernung der Luft abgeschlossen ist, in dem Venöse-Luft-Entfernungsfenster (74) eine Aufforderung an den Anwender anzuzeigen, dass er die manuell betätigbare Klemme (25) wieder öffnen soll.

10. Blutbehandlungsvorrichtung (2) nach Anspruch 9, **dadurch gekennzeichnet, dass** die Steuereinheit (68) eingerichtet ist, eine Blutbehandlungstherapie wieder aufzunehmen, wenn der Anwender auf dem Touch-Screen bestätigt hat, dass er die manuell betätigbare Klemme (25) geöffnet hat, und die Steuereinheit (68) von dem Luftdetektor (28) die Information erhält, dass sich keine Luft mehr in dem venösen Abschnitt (14) befindet.

11. Blutbehandlungsvorrichtung (2) nach Anspruch 10, **dadurch gekennzeichnet, dass** die Steuereinheit (68) eingerichtet ist
zu überprüfen, ob die manuell betätigbare Klemme (25) nach einer Wiederaufnahme der Therapie tatsächlich geöffnet ist, indem eine Druckänderung in dem extrakorporalen Kreislauf (4) überwacht wird, und
falls die manuell betätigbare Klemme (25) noch geschlossen ist, einen Alarm zum Öffnen der manuell betätigbaren Klemme (25) zu erzeugen.

## Claims

1. Blood treatment device (2) for use in blood treatment therapies, comprising:
an extracorporeal blood circuit (4), a dialyzer (6) and a dialysis fluid circuit (8), wherein the extracorporeal blood circuit (4) and the dialysis fluid circuit (8) are separated from each other via a membrane (10) provided in the dialyzer (6), via which blood can be filtered, and the extracorporeal blood circuit (4) has an arterial portion (12) upstream of the dialyzer (6) and a venous portion (14) downstream of the dialyzer (6);
an air detector (28) arranged in the venous portion (14) and configured to continuously monitor whether air is present in the venous portion (14);
a plurality of pumps (20, 36, 46, 56), including at least one blood pump (20) which is a pump in the arterial portion (12) and is configured to pump blood through the extracorporeal blood circuit (4);
a venous hose clamp (30), which is a hose clamp in the venous portion (14) and is configured to selectively clamp or release the venous portion (14);
a user interface (70) comprising a display (72) with a touch screen; and
a control unit (68), which is configured,
when it receives information from the air detector (28) that there is air in the venous portion (14), to stop the blood pump (20), to close the venous hose clamp (30), to raise an alarm, and to display on the display (72) a venous air removal window (74) displaying instructions to a user for removing the air in the venous portion (14) and displaying status reports about the removal of the air; and
to carry out an automatic removal of air from the venous portion (14) on a user-initiated basis,
**characterized in that**
a manually actuatable clamp (25) is provided in the venous portion (14) in addition to the venous hose clamp (30), by means of which a user can selectively clamp or release the venous portion (14).

2. Blood treatment device (2) according to claim 1, **characterized in that** the manually actuatable clamp (25) is arranged at the dialyzer outlet (23) in the venous portion (14) and thus upstream of the venous hose clamp (30).

3. The blood treatment device according to claim 1 or 2, **characterized in that** the control unit (68) is configured, when there is air in the venous portion (14), to display in the venous air removal window (74) an instruction for the user to close the manually actuatable clamp (25) in the venous portion (14).

4. Blood treatment device (2) according to claim 3, **characterized in that** the control unit (68) is configured, when there is air in the venous portion (14), to display in the venous air removal window (74) an air-removal button (76) which is actuatable by the user by pressing the touch screen.

5. A blood treatment device according to claim 4, **characterized in that** the control unit (68) is configured, when it detects that the air-removal button (76) has been pressed by the user, to check whether the manually actuatable clamp (25) is closed by monitoring a pressure change in the extracorporeal circuit (4), and to generate an alarm to close the manually actuatable clamp (25) if the manually actuatable clamp (25) is not closed.

6. Blood treatment device (2) according to claim 4 or 5, **characterized in that** the control unit (68) is configured, when it detects that the air-removal button (76) has been pressed by the user, to generate a negative pressure, which is less than a predefined value, in a venous air trap (26) arranged in the venous portion (14) by operating/ actuating a gauge/level control pump (33) and then to open the venous hose clamp (30) to automatically suck out air from the venous portion (14).

7. Blood treatment device (2) according to claim 6, **characterized in that** the predefined value is -50 mmHg.

8. Blood treatment device (2) according to claim 6 or 7, **characterized in that** the control unit (68) is configured, when it detects that air has been sucked out of the venous portion (14), to close the venous hose clamp (30) again and to indicate in the venous air removal window (74) that the removal of air is complete.

9. Blood treatment device (2) according to claim 8, **characterized in that** the control unit (68) is configured, when it detects that the removal of air is complete, to display (72) in the venous air removal window (74) a prompt to the user to open the manually actuatable clamp (25) again.

10. Blood treatment device (2) according to claim 9, **characterized in that** the control unit (68) is configured to resume a blood treatment therapy when the user has confirmed on the touch screen that he has opened the manually actuatable clamp (25), and the control unit (68) receives the information from the air detector (28) that there is no more air in the venous portion (14).

11. Blood treatment device (2) according to claim 10, **characterized in that** the control unit (68) is configured
to check whether the manually actuatable clamp (25) is actually open after a resumption of the therapy by monitoring a change in pressure in the extracorporeal circuit (4), and
if the manually actuatable clamp (25) is still closed, to generate an alarm to open the manually actuatable clamp (25).

## Revendications

1. Dispositif de traitement du sang (2) destiné à être utilisé dans des thérapies de traitement du sang comprenant :
une circulation sanguine extracorporelle (4), un dialyseur (6) et un circuit de dialysat (8), dans lequel la circulation sanguine extracorporelle (4) et le circuit de dialysat (8) sont séparés l'un de l'autre par le biais d'une membrane (10) prévue dans le dialyseur (6), par laquelle le sang peut être filtré, et la circulation sanguine extracorporelle (4) présente un segment artériel (12) en amont du dialyseur (6) et un segment veineux (14) en aval du dialyseur ;
un détecteur d'air (28), lequel est disposé dans le segment veineux (14) et conçu pour surveiller en continu le segment veineux (14), afin de déceler la présence d'air dans le segment veineux (14) ;
une pluralité de pompes (20, 36, 46, 56), y compris au moins une pompe à sang (20), laquelle est une pompe située dans le segment artériel (12) et est conçue pour pomper le sang à travers la circulation sanguine extracorporelle (4) ;
un collier de serrage veineux (30), lequel est un collier de serrage situé dans le segment veineux (14) et est conçu pour clamper ou libérer sélectivement le segment veineux (14) ;
une interface utilisateur (70) comprenant un écran (72) avec écran tactile ; et
un dispositif de commande (68), lequel est conçu
pour arrêter la pompe à sang (20), lorsqu'il reçoit une information en provenance du détecteur d'air (28) indiquant la présence d'air dans le segment veineux (14), pour fermer le collier de serrage veineux (30), pour émettre un signal d'alarme et pour afficher à l'écran (72) une fenêtre d'élimination de l'air dans le segment veineux (74), laquelle indique des instructions destinées à un utilisateur pour éliminer l'air présent dans le segment veineux (14) et des rapports d'état relatifs à l'élimination de l'air ; et
pour réaliser à l'initiative de l'utilisateur une élimination automatique de l'air du segment veineux (14),
**caractérisé en ce que**
dans le segment veineux (14) en plus du collier de serrage veineux (30), un clamp actionnable manuellement (25) est prévu au moyen duquel un utilisateur peut clamper ou libérer sélectivement le segment veineux (14).

2. Dispositif de traitement du sang (2) selon la revendication 1, **caractérisé en ce que** le clamp actionnable manuellement (25) est disposé dans le segment veineux (14) au niveau de la sortie du dialyseur (23) et donc en amont du collier de serrage veineux (30).

3. Dispositif de traitement du sang (2) selon la revendication 1 ou 2, **caractérisé en ce que** le dispositif de commande (68) est conçu pour afficher, lorsque de l'air se trouve dans le segment veineux (14), dans la fenêtre d'élimination de l'air dans le segment veineux (74) une instruction destinée à l'utilisateur lui indiquant qu'il doit fermer le clamp actionnable manuellement (25) dans le segment veineux (14).

4. Dispositif de traitement du sang (2) selon la revendication 3, **caractérisé en ce que** le dispositif de commande (68) est conçu pour afficher, lorsque de l'air se trouve dans le segment veineux (14), un bouton d'élimination de l'air (76) dans la fenêtre d'élimination de l'air dans le segment veineux (74), lequel peut être actionné par l'utilisateur en appuyant sur l'écran tactile.

5. Dispositif de traitement du sang (2) selon la revendication 4, **caractérisé en ce que** le dispositif de commande (68) est conçu pour surveiller, lorsqu'il détecte que le bouton d'élimination de l'air (76) a été enfoncé par l'utilisateur, si le clamp actionnable manuellement (25) est fermé, en surveillant une variation de pression dans la circulation extracorporelle (4), et pour générer un signal d'alarme pour fermer le clamp actionnable manuellement (25) lorsque le clamp actionnable manuellement (25) n'est pas fermé.

6. Dispositif de traitement du sang (2) selon la revendication 4 ou 5, **caractérisé en ce que** le dispositif de commande (68) est conçu pour produire, lorsqu'il détecte que le bouton d'élimination de l'air (76) a été enfoncé par l'utilisateur, une pression négative, laquelle est inférieure à une valeur prédéfinie, dans un élément collecteur d'air veineux (26) disposé dans le segment veineux (14) en actionnant/activant une pompe de régulation de niveau (33), et pour ouvrir consécutivement le collier de serrage veineux (30), pour évacuer automatiquement l'air du segment veineux (14).

7. Dispositif de traitement du sang (2) selon la revendication 6, **caractérisé en ce que** la valeur prédéfinie est 50 mmHg.

8. Dispositif de traitement du sang (2) selon la revendication 6 ou 7, **caractérisé en ce que** le dispositif de commande (68) est conçu pour refermer, lorsqu'il détecte que de l'air a été aspiré du segment veineux (14), le collier de serrage veineux (30) et pour afficher dans la fenêtre d'élimination de l'air du segment veineux (74) que l'élimination de l'air est terminée.

9. Dispositif de traitement du sang (2) selon la revendication 8, **caractérisé en ce que** le dispositif de commande (68) est conçu pour afficher, lorsqu'il détecte que l'élimination de l'air est terminée, une invitation à l'utilisateur dans la fenêtre d'élimination de l'air dans le segment veineux (74) indiquant qu'il doit rouvrir le clamp actionnable manuellement (25).

10. Dispositif de traitement du sang (2) selon la revendication 9, **caractérisé en ce que** le dispositif de commande (68) est conçu pour reprendre la thérapie de traitement du sang, lorsque l'utilisateur a confirmé sur l'écran tactile, qu'il a ouvert un clamp actionnable manuellement (25), et que le dispositif de commande (68) reçoit l'information du détecteur d'air (28) indiquant qu'il n'y a plus aucune présence d'air dans le segment veineux (14).

11. Dispositif de traitement du sang (2) selon la revendication 10, **caractérisé en ce que** le dispositif de commande (68) est conçu
pour surveiller si le clamp actionnable manuellement (25) est réellement ouvert après une reprise de la thérapie, en surveillant une variation de pression dans la circulation extracorporelle (4), et
dans le cas où le clamp actionnable manuellement (25) est encore fermé, pour générer un signal d'alarme pour ouvrir le clamp actionnable manuellement (25).
